# EUROPEAN PATENT APPLICATION

(11) **EP 1 712 198 A2**
(43) Date of publication of application: **18.10.2006**
(21) Application number: 05103527.7
(22) Date of filing: 28.04.2005
(51) Int. Cl.: A61B 17/82

(54) **Bone fixation device for surgical operation, cutting device and band head for the same**

(30) Priority: 13.04.2005 JP 2005115566
(71) Applicant: Padl Inc., Osaka-shi, Osaka 532-0011, Osaka (JP)
(72) Inventor: Kizuka, Takeshi, 532-0011, Osaka (JP); Kawasaki, Yoshinori, 836-0096, Fukuoka (JP); Kinoshita, Shousaku, 837-0922, Fukuoka (JP)
(74) Representative: Fleuchaus, Michael A.

(57) **Abstract**

Provided is a bone fixation band which can be easily handled, enables to securely fasten the bone, and also to prevent the post-operative inconveniences by forming the head part as thin as possible. The band head of the bone fixation device is constituted of a plate being bent in roughly an N-letter shape. The plate comprises three through-holes arranged in the extended direction of the band. The band is passed through into the through-hole just like sawing and then is folded back to pass through again into the through-hole and fixed. A sharp and curved needle is provided at the tip of the band for easily being set around the bone to be a fixation target and also for making a hole in the bone so that the band can be easily passed through.

## Description

### BACKGROUND OF THE INVENTION

### 1) Field of the Invention

The present invention relates to a bone fixation device for a surgical operation and, more specifically, to a bone fixation device for fixing once-separated parts of a sternum and/or opened ribs in the operation for the inside of the sternum and the ribs to be restored as before.

### 2) Related Art

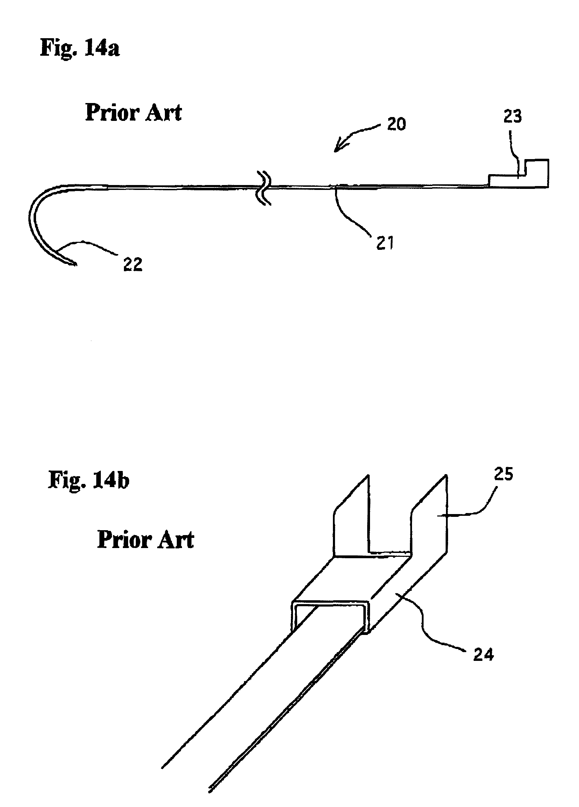

For example, in the case of a cardiac operation, in which the sternal cartilages are completely opened vertically for performing the operation inside a sternum, a bone fixation band is used for restoring the separated parts of the sternum back in place upon closing the chest (A product catalog "Thoracic Cerclage Bands" (Approved No. 21100BZY00311000), products of Tran Systeme S. A., imported and distributed by NIPPON BXI, Inc.). Fig. 14 is an illustration showing a conventional bone fixation band. As shown in Fig. 14(a), a bone fixation band 20 is comprised of a long and flat band 21, a needle 22 provided at one end of the band 21, and a head 23 provided at the other end of the band 21. The end of the needle 22 is curved so that the band 21 can be set behind a bone and pulled through to the front again. For each structural element, biocompatible stainless steel is used. As shown in Fig. 14(b), the head 23 comprises an angular cylindrical insertion part 24 and a pair of wings 25.

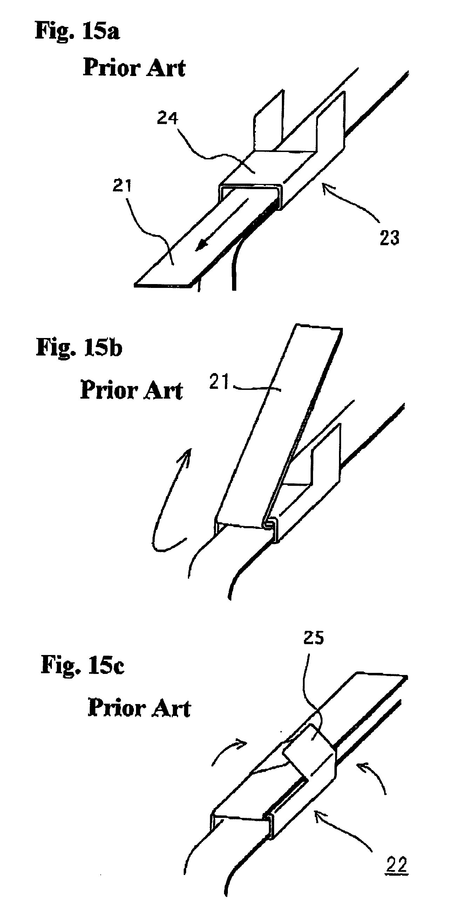

Fig. 15 is an illustration showing the method of using the conventional bone fixation device. The bone fixation band is set around the target bone with the needle 22, and the needle 22 is cut from the band 21 with a cutter or the like. Then, as shown in Fig. 15(a), the tip of the band is inserted into the insertion part 24 of the head 23. After fully pulling the band 21 which emerged from the opposite side of the head 23, the band 21 is bent towards the opposite side as shown in Fig. 15(b). Then, as shown in Fig. 15(c), the pair of the wings 25 of the head 23 are pushed down onto the band 21 so that the band 21 is not loosened. The extra portion of the head 23 is then cut off with a cutter or the like thereby completing the fixation.

However, when using the above-described bone fixation band, it is necessary to push down the wings 25 on both sides with a pair of pliers or the like after folding back the band 21, hence making it a complicated and delicate work with a certain level of necessary skill involved as well. The treatment also takes time. Furthermore, the insertion part 24 of the head 23 is made up as high as 5 mm or more for keeping its angular cylindrical shape. Due to this height, there is a possibility that a patient may complain of an uncomfortable feeling or pain after closing the chest. Furthermore, the insertion part 24 has space for inserting the band 21, so that infections may be caused when body fluids or tissues are caught in the space after fixing the bone.

### SUMMARY OF THE INVENTION

Therefore, the object of the present invention is to provide a bone fixation band which can be easily handled, securely fasten a bone, and prevent post-operative inconveniences by making up a head part as thin as possible.

In order to achieve the foregoing object, the present invention provides a bone fixation device used for fixing a bone in a surgical operation, and is comprised of a long strip-shaped band and a band head provided at an end of the band, in which the band head is constituted of a single plate having two or more through-holes arranged in an extended direction of the band.

In the bone fixation device of the present invention, it is effective that the band head is curved or bent so that the band can be easily inserted into the through-holes successively.

In the bone fixation device of the present invention, it is effective that the band and/or the band head are/is made of a thin metal material and preferably of stainless steel.

In the bone fixation device of the present invention, it is effective that at least the through-hole of the band head on the attaching side of the band is in a circular shape or an oval shape.

In the bone fixation device of the present invention, it is effective that a curved needle is provided at the free end of the band. In this case, it is effective that the needle is entirely flat, has a sharply pointed tip, and at least a part of it is of the same width as that of the band or slightly wider.

Furthermore, the present invention relates to a cutting tool for cutting the band of the bone fixation device, and is comprised of a guide groove for guiding the band, a blade for cutting the band, and a handle for handling the blade, in which the blade has a curved shape for cutting the tip of the band into a round shape.

Moreover, the present invention relates to a band head for use in a bone fixation device which is used for fixing a bone in a surgical operation, and is comprised of a single plate which has two or more through-holes arranged in line.

In this band head, it is effective that the plate is curved or bent so that the band can be easily inserted into the through-holes successively and at least one or more of the through-hole of the band head is in a circular shape or an oval shape.

For using the bone fixation device of the present invention, the band is set around the bone to be the fixation target, the tip of the band is inserted into the through-holes provided in the band head just like sawing, is folded back after being fully pulled, and is inserted into the through-hole again as necessary for completing the fixation. Since the band head is constituted of a single plate, the thickness of the fixed part can be remarkably thinned compared to the conventional case. Thus, it enables to prevent patients from experiencing an uncomfortable feeling or a pain caused by a contact with tissues after the operation. Furthermore, unlike the conventional device, the band head is not in an angular cylindrical shape, so that the possibility of infections caused by body fluids or tissues caught in the space can be dramatically reduced. Moreover, unlike the conventional device, it is not necessary to push down the wings with a pair of pliers, so that it can be easily treated. Moreover, the device can be manufactured at a far lower cost compared to the conventional bone fixation device.

Furthermore, the band head is curved or bent to easily pass the band through the through-holes successively, so that the surgery becomes easy and the required time can be shortened. When the bone fixation device is constituted of stainless steel of high quality, the bone can be securely fixed over a long period of time by folding back the band using the plasticity of the metal.

Moreover, the curved needle is provided at the tip of the band, so that it becomes easier to set the band around the bone. In addition, this needle is entirely flat, has a sharply pointed tip, and at least a part of it is of the same width as that of the band or slightly wider. Thus, it becomes possible to pass the band through the bone by penetrating it with the needle. For example, when a hole is made in an episternum to pass a band through and fix the separated sternum upon closing a patient's chest in a thoracic operation, the stress to be given to the episternum and the capillary inside the episternum can be minimized.

Further, by using the cutting device for cutting the tip of the band into a round shape, damages on the tissues which are caused by the tip of the band during or after the surgery can be prevented.

### BRIEF DESCRIPTION OF THE DRAWINGS

Preferred embodiments of the present invention will be described in detail hereinafter by referring to accompanying drawings:
Fig. 1 is an illustration showing the configuration of the first embodiment of the bone fixation device according to the present invention;
Fig. 2 is a perspective view showing the configuration of a needle 12;
Fig. 3 is a perspective view showing the configuration of a band head 13 shown in Fig. 1;
Fig. 4 is a perspective view showing the configuration of a supporting device for using the bone fixation device;
Figs. 5 (a and b) are illustrations showing the configuration of a cutting device for using the bone fixation device;
Fig. 6 shows an example in which the bone fixation device is applied to the sternum;
Figs. 7 (a to d) show the method of using the bone fixation device shown in Fig. I;
Figs. 8 (e to h) show the method of using the bone fixation device 1 shown in Fig. 1;
Figs. 9 (a to c) show the method of using a bone fixation device according to the second embodiment of the present invention;
Figs. 10 (d to f) show the method of using the bone fixation device according to the second embodiment of the present invention;
Fig. 11 is an illustration showing the configuration of a bone fixation device according to the third embodiment of the present invention;
Figs. 12 (a and b) are illustrations showing the configuration of a bone fixation device according to the fourth embodiment of the present invention;
Figs. 13 (a and b) are illustrations showing the configurations of a band head in still other embodiments;
Figs. 14 (a and b) show the configuration of a conventional bone fixation device; and
Figs. 15 (a to c) show the method of using the conventional bone fixation device.

### DETAILED DESCRIPTION OF THE PREFERRED EMBODIMENT

Preferred embodiments of the present invention will be described in detail by referring to accompanying drawings.

### (FIRST EMBODIMENT)

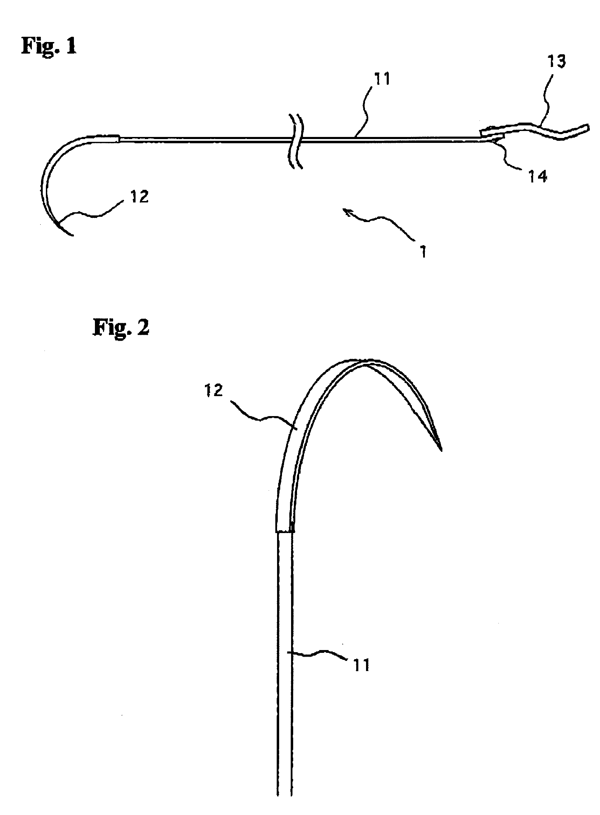

Fig. 1 is an illustration showing the configuration of the first embodiment of the fixation device according to the present invention. As shown in Fig. 1, a bone fixation device 1 comprises a long strip-shaped band 11, a needle 12 provided at one end of the band 11, and a band head 13 provided at the other end of the band 11. Each of the elements is made of biocompatible metallic material and, desirably, of the non-ferritic (non-magnetic) high quality stainless steel. As an example of such metal, stainless steel 316LVM (product name), a product of Sandvik AB, can be preferably used.

The band 11 has flexibility and plasticity and it is formed to have a length of about 150 - 400 mm, a width of about 1.5 - 12.0 mm, and a thickness of about 0. 1 - 1.0 mm, for example. The needle 12 is curved so that it can be set behind the bone and then pulled through to the front again. It is formed in a length of about 40 - 60 mm, a width of about 1.5 - 3.5 mm, and a thickness of about 1.0 - 2.0 mm. Fig. 2 shows a needle 12 of the embodiment. As shown in the drawing, the needle 12 is entirely flat, has a sharply pointed tip, and at least a part of it is of the same width as that of the band 11 or slightly wider. With this configuration, the band 11 can be passed through by inserting the needle 12 into a part of the episternum or the like, for example. As for the needles 12, the dimensional shape, the degree of the curvature and the like are to be determined appropriately in accordance with the area to be applied. For example, a round needle called "round point" may be used in an area which is soft and easy to insert the needle, and a tapered cutting needle with a triangular pyramid point in the embodiment is used for an area where it is hard to insert the needle.

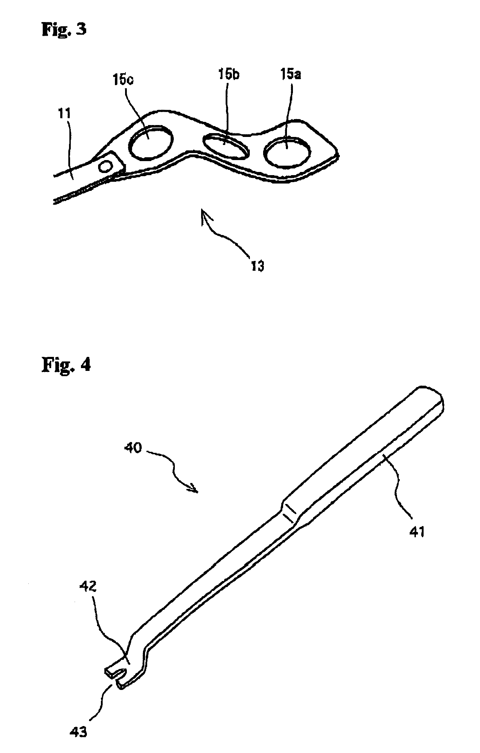

The band 11 is fixed to the band head 13 through a caulking pin or rivets 14 and, as shown in Fig. 1, is slightly curved or bent in an N-letter shape, The head 13 is formed in a size of about 7 - 50 mm in length, 4 - 15 mm in width, and 0.2 - 2.0 mm in thickness before being bent. Fig. 3 is a perspective view showing the configuration of the head 13. As shown in the drawing, the head 13 has a shape of rectangular plate with round edges whose one end is narrowed to the width of the band 11. The head 13 is provided with three through-holes 15a, 15b, and 15c arranged at almost equal intervals in the extended direction of the band 11. Furthermore, the band head 13 is curved or bent at an obtuse angle between with the adjacent through-holes 15 and, as a whole, it forms roughly an N-letter shape. The through-holes 15 are circles with the diameter which is the same as the width of the band 11 or slightly larger (for example, about 1.5 - 12 mm), and the distance from the center of one through-hole to the center of the next through-hole is the radius of the hole or shorter (for example, about 2 - 15 mm). The N-letter shape of the band head makes easier for the band 11 to pass through as described above, and can prevent the band 11 from passing the through-holes 15 in the wrong order.

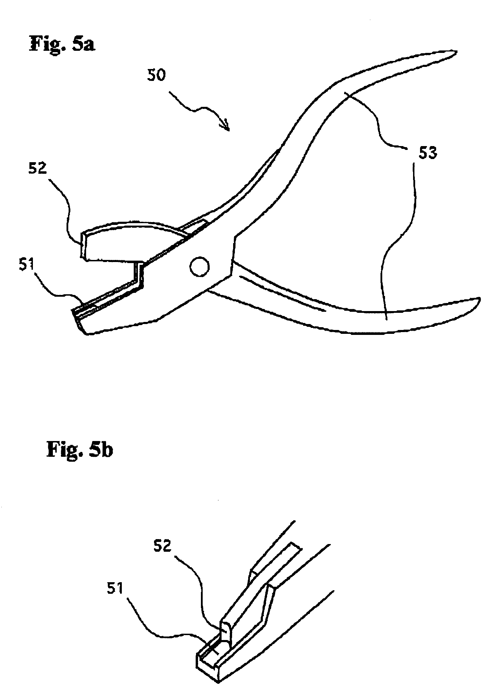

A device used for using the bone fixation device 1 will be described by referring to Fig. 4 and Fig. 5. Fig. 4 shows a supporting device 40 and Fig. 5 shows a cutting tool 50. The supporting device 40 is a device for pushing down the band head 13 when the band 11 taken out from the through-holes 15 is pulled. It comprises a grip 41 and a supporting end 42 being bent in a crank shape. A U-shape groove 43 in a width slightly wider than the band 11 is provided on the tip of the supporting end 42. Through pushing down the band head 13 with applying the band to the U-shape groove 43 makes it possible to pull the band 11 without loosening the band head 13 from the bone.

Fig. 5(a) is a perspective view showing the overall configuration of the cutting tool 50, and Fig. 5(b) is an enlarged view of the tip of the tool. As shown in the drawings, the cutting tool 50 has the configuration of a pair of pliers, and is comprised of a guide groove 51 for guiding the band 11 which is of the same width as that of the band 11 or slightly wider, a blade 52 for cutting the band, and a handle 53 for handling the blade. The guide groove 51 is in communication with the handle 53, and the band 11 can be inserted into the guide groove 51 to be pulled out at some length toward the handle 53. As shown in Fig. 4(b), the blade 52 has a curved shape, so that it can cut the band 11 in a round cut face.

The dimensions and the shapes of the above-described structural elements can be appropriately altered according to the condition of embodiment such as the area of to be applied the bone fixation device 1.

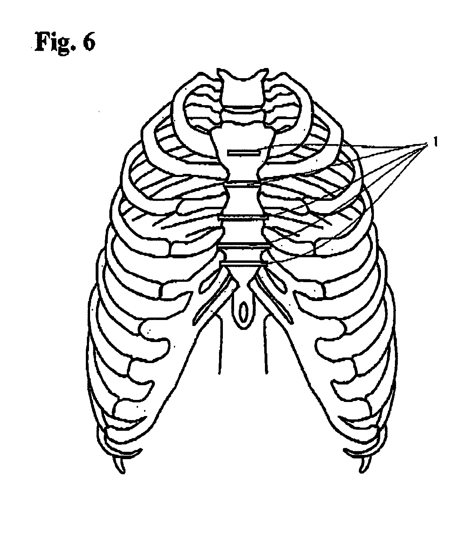

The method of using the bone fixation device 1 constituted in the manner as described above will be described in the followings, Fig. 6 is an illustration showing an example of the area to be applied the bone fixation device 1 according to the present invention. The bone fixation device 1 of the present invention is used for fixing the separated sternal bones by bringing them together when, as shown in Fig. 6 for example, the sternum is completely separated vertically in a operation for the inside of the sternum. That is, the vertically separated sternal bones are unified again, and a plurality of bone fixation devices 1 is wrapped around the bones to fix. Furthermore, in the episternum on the upper part of the strenum, it is fixed with the band 11 passed through by being penetrated with the needle 12, In this case, the tip of the needle 12 is sharply pointed and has the same or slightly wider width of the band 11, so that it enables to suppress damages on the capillary inside the bones compared to the case where the band 11 is inserted by opening a hole in the bone through a spindle or the like. Since the band 11extends in the direction of inserting the needle 12, it is necessary to pay an attention to the inserting direction of the needle 12 while in operation.

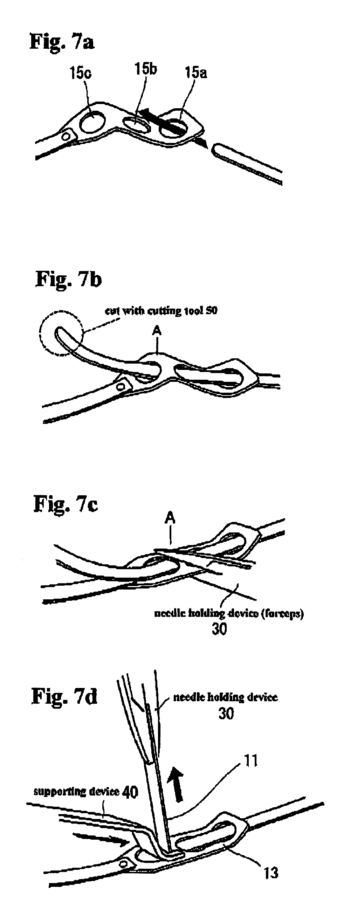

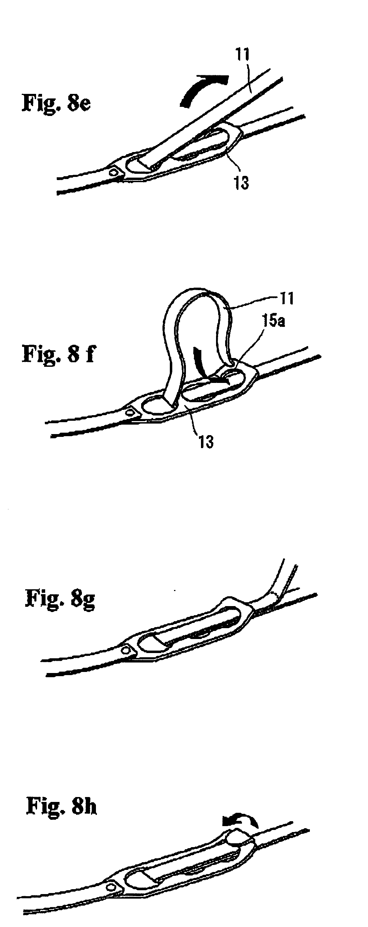

Fig. 7 and Fig. 8 are illustrations showing an example of the method for using the bone fixation device 1. In surgery, at first, the needle 12 is set around the bone to be the target of the fixation, or, for the episternum, the needle 12 is inserted into the episternum to pass the band 11 through. At this time, it is necessary to pay attention to the direction of the band so that the surface side (the top side in Fig. 1) of the band head 13 comes face up, Furthermore, it is necessary to mind that the band 11 is not twisted while handling the needle.

After setting the band 11 around the bone, the needle 12 is cut off (not shown) from the band 11 using the cutting tool 50 or other devices. After confirming that there is no twist in the band 11, the free end of the band 11 is pulled out from the backside of the first through-hole 15a. Then, it is inserted into the second through-hole 15b, and pulled out again from the third through-hole 15c, as shown in Figs. 7(a) and 7(b). In this case, since the band head 13 is roughly in an N-letter shape, the direct insertion of the tip of the band 11 in the through-holes makes it possible to pass through the three adjacent through-holes 15a-15 c. In addition, the band 11 can be inserted to the surface side or backside in the right order.

Next, as shown in Fig. 7(c), protrusion part A between the second through-hole 15b, and the third through-hole 15c is flattened by using forceps 30 such as the needle holding device. Accordingly, the part of the band head 13 which contacts with the bone becomes flattened, so that the pressure applied by the head 13 is dispersed, and the inflammation caused by the invasion of tissues or body fluid into space between the bone and the band head 13 can be prevented. Next, as shown in Fig. 7(d), the band 11 is fully pulled with the needle holding device 30 while pressing the bottom part of the band head 13 with the supporting device 40 for ensuring that the bones are closely contacted with each other by the fixation device 1.

Next, as shown in Fig. 8(e), the band 11 is folded back to the opposite side and the tip of the band 11 is inserted into the through-hole 15a as shown in Fig. 8(f). Furthermore, as shown in Fig. 8(g), the extra portion is cut off by the cutting device 50 and the tip of the band 11 is further folded back to the front. The band 11 is made of a metallic material and, by folding it back, it is more securely fixed and not to be come off from the through-hole later on. At last, the tip portion of the band head 13 is pressed by the supporting device 40 and the like to be flattened. The necessary areas are fastened by the bone fixation device 1 in the manner as described above and, thereafter, the tissues around the bone are sutured up according to an ordinary procedure to complete the operation.

By using the bone fixation device 1 as described above, the bones can be securely fixed. Furthermore, the three through-holes 15a - 15c have circular shapes, so that each of the holes 15 has two contact points with the strip-shaped band 11 which pass through them. Accordingly, combined with the elasticity and the flexibility of the metal band, the two contact points acts for tight fixation of the band. Furthermore, the band head 13 is constituted of a thin plate and the overall height is kept low even in the fixed state. Thus, patients feel no post-operative pain caused by the contact of the band head 13 with their tissues. Moreover, little space is generated between the band 11 and the band head 13 in the fixed state, so that no inflammation is caused by the tissues invaded inside the device. In addition, the cutting tool 50 cuts the part of the band 11 in a semicircle shape, so that no tissues are damaged from the cut part.

### (SECOND EMBODIMENT)

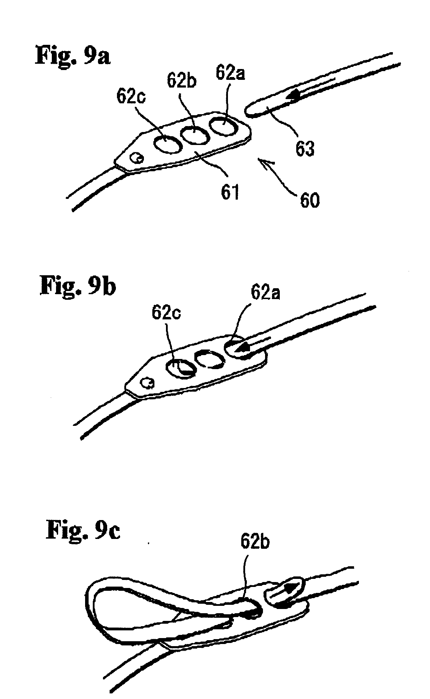

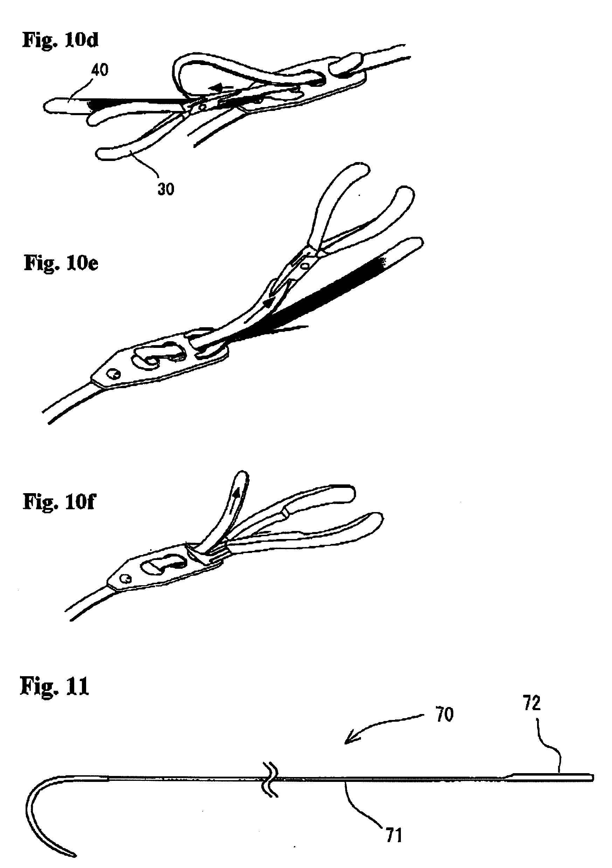

Next, the second embodiment of the bone fixation device according to the present invention will be described. Fig. 9 and Fig. 10 are illustrations for describing a bone fixation device 60 according to the embodiment and the using method of the device. In the embodiment, a band head 61 of the band 60 is not in roughly an N-letter shape and is constituted of a single flat plate comprising three through-holes 62a, 62b, and 62c. Other configurations are the same as those of the first embodiment shown in Fig. 1.

The band 63 is placed around the bone by using the bone fixation device 60 of the embodiment, and the needle is cut off from the band 63 by using a device such as a cutter or the cutting tool 50 (not shown). After confirming that there is no twist in the band 63, as shown in Figs. 9(a) - 9(b), the free end of the band is inserted into the first through-hole 62a from over the head 61 and is pulled out to the top from the third trough-hole 62c. Then, as shown in Fig. 9(c), the band 63 is turned over to be inserted into the second through-hole 62b, and is pulled out to the surface again from the first through-hole 62a.

Next, as shown in Fig. 10(d), the band is pulled by the needle holding device 30 while pressing the bottom of the band in the third through-hole 62c by the supporting device 40. Furthermore, as shown in Fig. 10(e), the end of the band is pulled by the needle holding device 30 by pressing the bottom of the band in the first through-hole 62a by the supporting device 40. After confirming that the bone is fixed in this manner under a desirable state, as shown in Fig. 10(f), the extra portion of the band 63 coming out from the first through-hole 62a is cut off thereby completing the fixation. After that, the tissues around the bone are sutured up through an ordinary procedure thereby completing the operation. The cutting of the band 63 may be performed by the cutting device 50 shown in Fig. 5.

In the embodiment, the metallic band 63 is folded back after pulling out from the third through-hole 62c, and the part of the band which is then inserted into the first through-hole 62a from the second through-hole 62b is constantly pushed down. Thus, the band 63 is not to be come off from the through-holes after the fixation. Thus, different variations on the insertion manner of the band into the through-holes may be devised.

### (THIRD EMBODIMENT)

Fig. 11 is a side view showing the configuration of the third embodiment of the bone fixation device according to the present invention, As shown in Fig. 11, in a bone fixation device 70 of the present invention, a band 71 and a band head 72 are integrally molded. In this bone fixation device 70, the head 72 is formed to be thicker than the band 71 for increasing the strength. Other configurations are the same as those of the bone fixation device 60 of the second embodiment. With this configuration, it becomes unnecessary to fasten the band and the band head by the rivet. Thus, the number of components can be decreased and the height of the bone fixation device 70 can be lowered for the height of the rivet. As the method for manufacturing the bone fixation device 70, there may be a method in which a metal plate with the thickness of the band head 72 is prepared and is cut into a desired shape, and only the part for the band 71 is then stretched thin.

### (FOURTH EMBODIMENT)

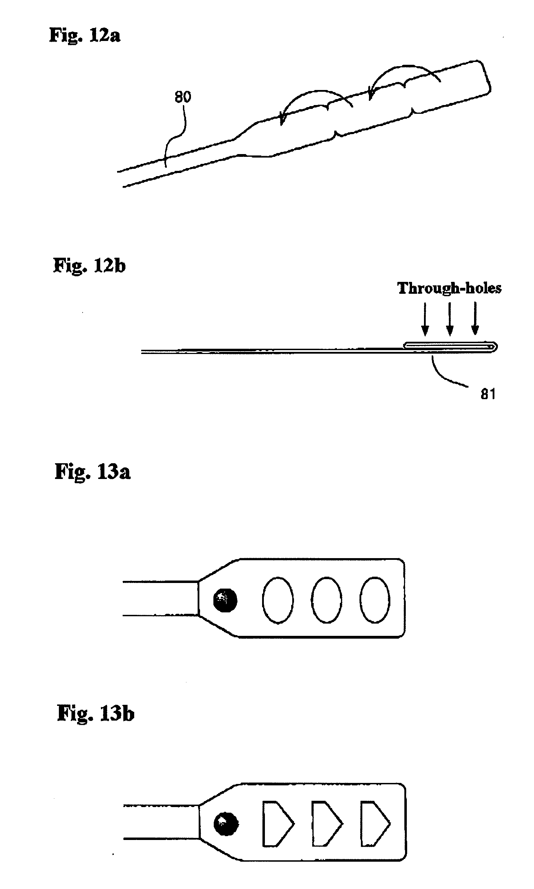

Fig. 12 is an illustration showing the configuration of the fourth embodiment of the bone fixation device. In a bone fixation device 80 shown in Fig. 12, a band and a band head are also integrally molded so that the rivet conjunction between the both elements becomes unnecessary. In the embodiment, as shown in Fig. 12(a), a metal plate with the thickness of the band is cut into a shape in which a plurality of the band head contours are jointed to be folded back and pressed as shown in Fig. 12(b). Through-holes are then made to complete a band head 81. With this configuration, it is possible to obtain the band head with the desirable strength at a relatively low cost. In the embodiment shown in Fig. 12, the metal plate is folded three times for making up the band head. However, it may be folded twice, or four times or more.

In the embodiments described above, the through-holes are in a circular shape. However, the through-holes may be in other shapes as long as the belt can be preferably fastened. For example, the through-holes may be in an oval shape as shown in Fig. 13(a) or may be in a roughly triangular shape as shown in Fig. 13(b). With these configurations, the through-hole comes in contact with the two contacts at the edge of the belt when the belt is inserted. Thus, together with the flexibility of the belt, it is possible to securely fasten the belt.

Although it is desirable to form all the three belt through-holes in the above-described shapes, only the third through-hole (the through-hole 15c in Fig. 2) which is the nearest to the belt may be in the above-described shape and the other holes may be in any other shapes (for example, rectangular and the like). Since the strongest force is imposed upon the folded part of the belt, the necessary and sufficient fixation strength can be obtained by making at least one through-hole in the folded part in the above-described shape.

Furthermore, three through-holes are provided in the bone fixation devices of all the above-described embodiments, however, the device may be provided with two, four or more band-through-holes. When there are two through-holes, the band is pulled out from the through-hole situated in a attaching side of the band, is then folded back to be inserted into another through-hole and is fixed. When there are four through-holes, there may be different variations, and by folding back the band pulled out from a hole and inserting it to another hole, the band can be securely fixed. The band head is in a flat plate body in the above-described second embodiment, however, for the bone fixation device especially used for turning around thin bones, the band head may be made up in a plate body which is previously curved along the outer shape of the bone. Furthermore, although the bone fixation device comprises the needle in the above-described embodiments, the needle is not the essential component and the bone fixation device may be constituted with only the band and the band head. As described above, the dimensional shapes and the degree of the curvature of the needle are determined appropriately according to the condition of embodiment such as the applied area and the like.

In the case of cardiac operation, for example, in which the sternum is completely separated vertically for performing an operation inside the sternum, five to six bone fixation devices of the present invention are disposed around the separated sternal bones to join and fix them. Moreover, when an internal operation is performed by opening the ribs from the side part of a human body, two to three bone fixation devices of the present invention are used for bringing the separated ribs together to be fixed. In addition, the bone fixation device 1 can be preferably used for fixing and reinforcing the cranial bones, thighbones and the like.

## Claims

1. A bone fixation device used for fixing bones in a surgical operation, comprising a long strip-shaped band and a band head provided at an end part of said band, wherein said band head is constituted of a single plate having two or more through-holes arranged in an extended direction of said band.

2. The bone fixation device according to claim 1, wherein said band head is curved or bent so that said band easily passes through into said through-holes successively.

3. The bone fixation device according to claim 1 or 2, wherein said band and/or said band head are/is made of thin metal material and, preferably, of stainless steel.

4. The bone fixation device according to any one of claims 1 to 3, wherein at least the through-hole of said band head on an attaching side of said band is in a circular shape or an oval shape.

5. The bone fixation device according to any one of claims 1 to 4, wherein a curved needle is provided at a free end part of said band.

6. The bone fixation device according to claim 5, wherein said needle is entirely flat, has a sharply pointed tip, and at least a part of the needle is formed in a same width as that of said band or slightly wider.

7. A cutting device for cutting said band of said bone fixation device according to any one of claims 1 to 6, comprising: a guide groove for guiding said band; a blade for cutting said band; and a handle for handling said blade; wherein said blade has a curved shape for cutting the tip part of said band into a round shape.

8. A band head for a bone fixation device used for fixing bones in a surgical operation, comprising a single plate which has two or more through-holes arranged in line,

9. The band head according to claim 8, wherein said plate is curved or bent so that said band easily passes through into said through-holes successively.

10. The band head according to claim 8 or 9, wherein at least one or more of the through-hole of said band head is in a circular shape or an oval shape.
